# EUROPÄISCHE PATENTSCHRIFT

(11) **EP 2 916 912 B1**
(45) Veröffentlichungstag und Bekanntmachung des Hinweises auf die Patenterteilung: **18.01.2017**
(21) Anmeldenummer: 13791756.3
(22) Anmeldetag: 30.10.2013
(51) Int. Cl.: A61N 5/10

(54) **VERFAHREN ZUR BESTRAHLUNGSPLANUNG**
METHOD FOR IRRADIATION PLANNING
PROCÉDÉ PERMETTANT D'ÉTABLIR DES PLANS D'IRRADIATION

(30) Priorität: 12.11.2012 DE 102012110864
(43) Veröffentlichungstag der Anmeldung: 16.09.2015
(73) Patentinhaber: GSI Helmholtzzentrum für Schwerionenforschung GmbH, 64291 Darmstadt (DE)
(72) Erfinder: GEMMEL, Alexander, 91056 Erlangen (DE); BERT, Christoph, 91080 Uttenreuth (DE)
(74) Vertreter: Blumbach Zinngrebe
(86) Internationale Anmeldenummer: PCT/EP2013/072690
(87) Internationale Veröffentlichungsnummer: WO 2014/072213

(56) Entgegenhaltungen:
- WO-A1-2010/043340
- DE-A1-102008 027 485
- DE-A1-102009 033 297

## Beschreibung

### Gebiet der Erfindung

Die Erfindung betrifft ein Verfahren und eine Vorrichtung zur Bestrahlungsplanung sowie eine Beschleunigereinrichtung mit einem Partikelstrahl.

### Hintergrund der Erfindung

Die Tumortherapie mit schweren Ionen hat sich im Laufe der letzten Jahrzehnte zu einem etablierten Verfahren zur Behandlung von Gewebe, insbesondere von Tumorerkrankungen, entwickelt. Die dabei gewonnenen Erfahrungen werden jedoch auch in rein technischen Gebieten angewendet.

Gemeinsames Merkmal aller bekannten Verfahren ist hierbei, dass ein von einer Beschleunigeranlage bereitgestellter gebündelter Partikelstrahl mittels eines Hochenergiestrahltransportsystems zu einem oder mehreren Bestrahlungs- oder Behandlungsräumen geleitet wird. In dem Bestrahlungsraum wird ein zu bestrahlendes Zielvolumen positioniert und mit dem Partikelstrahl bestrahlt.

Es ist bekannt, dass sich ein zu bestrahlendes Zielvolumen bewegen kann. Beispielsweise kann in dem Zielvolumen ein Lungentumor angeordnet sein, der bei einem Atemzug des Patienten bewegt wird. Zur Untersuchung des Einflusses der Bewegung auf den Behandlungserfolg der Partikeltherapie kann die Bewegung aber auch mittels als Phantomen bezeichneten, nicht lebenden Modellkörpern nachgebildet werden.

Eine besondere Herausforderung im Rahmen der Partikeltherapie ist es, eine möglichst homogene Verteilung der im Gewebe deponierten Strahlungsdosis zu erreichen. Ein Grund, weshalb eine homogene Dosisverteilung im Zielvolumen von besonderem Interesse ist, ist die Tatsache, dass die Zellen des im Zielvolumen befindlichen Tumors erst ab einer Schwelldosis mit ausreichender Sicherheit sterben, zugleich aber eine übermäßige Belastung des umliegenden gesunden Gewebes verhindert werden soll. So ist es nach wie vor schwierig, bei Bestrahlungsverfahren, bei denen eine Vielzahl von Einzelbestrahlungsdosen sukzessive an verschiedenen Zielpunkten im Zielvolumen deponiert werden soll, also bei einem gescannten Partikelstrahl, schwer, diese gewünschte homogene Dosisverteilung im Zielvolumen zu erreichen, wenn sich das Zielvolumen während der Bestrahlung bewegt. Die Verbesserung der Homogenität der Dosisverteilung im Zielvolumen ist daher nach wie vor Stand der Forschung.

Beispielsweise ist es bei einem gescannten Partikelstrahl eine Möglichkeit, die zu applizierende Strahlungsdosis auf mehrere Durchgänge zu verteilen, was als "Rescanning" bezeichnet wird. Hierbei werden die Zielpunkte des Zielvolumens mehrfach angefahren, so dass die zu applizierende Gesamtdosis sukzessive durch mehrere, wiederholt applizierte Einzeldosen während der Rescanning Durchgänge aufgebaut wird. Das wiederholte Anfahren der Zielpunkte mit Einzeldosen ermöglicht ein statistisches Mitteln über die Einzeldosen, wodurch möglicherweise fehlerhaft deponierte Dosen statistisch betrachtet gemittelt werden können. Bewegungen des Zielvolumens können so zumindest teilweise kompensiert werden.

Allerdings wird hierbei jeder Zielpunkt vielfach mit einer entsprechend verminderten Teildosis angefahren, wodurch das Bestrahlen mittels Rescanning eine erhebliche zusätzliche Zeitdauer beanspruchen kann, da die Extraktionsrate gemäß der verminderten Teildosis herabgesetzt werden muss. Es ist Stand der Technik, dass ein längerdauernder Bestrahlungsprozess in Kauf genommen werden muss, um die Homogenität der Dosisverteilung und, im Falle einer Therapiebestrahlung, somit den Behandlungserfolg zu erhöhen.

Des Weiteren ist es bekannt, die Bewegung des Zielvolumens zu verfolgen und zur Berechnung der Einzeldosen zu berücksichtigen.

Der für die Erfindung relevante Stand der Technik kann in den Schriften DE 10 2008 027485 A1, WO 2010/043340 A1 und DE 10 2009 033297 A1 gefunden werden.

### Allgemeine Beschreibung der Erfindung

Die Erfindung hat sich daher die Aufgabe gestellt, ein Verfahren bereit zu stellen, welches die Bestrahlungsplanung eines Zielvolumens vereinfacht und dabei die vorgenannten Nachteile des Standes der Technik mindert oder beseitigt.

Die Erfindung soll ferner die Zeitdauer der gesamten Behandlung bzw. Dosisapplikation verringern.

Eine weitere Aufgabe der Erfindung ist es, das Bestrahlungsergebnis robuster gegenüber Veränderungen, insbesondere Bewegungen, des Zielvolumens zu machen.

Die Aufgabe der Erfindung wird durch den Gegenstand der unabhängigen Ansprüche gelöst. Vorteilhafte Weiterbildungen der Erfindung sind in den Unteransprüchen definiert.

Die Bestrahlungsplanung eines in einem Körper angeordneten Zielvolumens, die Schritt für Schritt den Bestrahlungsplan erstellt, wird mit der vorliegenden Erfindung anhand der folgenden Schritte festgelegt:

Zunächst wird ein Zielvolumen festgelegt, welches typischerweise in einem Körper angeordnet ist. Das Zielvolumen kann dabei in einem nicht-lebenden Körper, wie beispielsweise einer Materialprobe, einem Phantom oder einem Versuchsaufbau angeordnet sein oder aber lebendes Material wie beispielsweise Zellproben oder Versuchstiere umfassen. Insbesondere befinden sich in dem Zielvolumen Tumorzellen. Das Zielvolumen wird in eine Vielzahl einzeln anfahrbarer Zielpunkte unterteilt. Mit anderen Worten wird in das Zielvolumen ein Punktraster aus Zielpunkten mit definierten Punktabständen gelegt.

Den Zielpunkten wird darauf hin jeweils eine Solldosis, d.h. der Plan- oder Sollwert der an dem jeweiligen Zielpunkt zu deponierenden Bestrahlungsdosis, zugeordnet. Mit anderen Worten wird in den Bestrahlungsplan die Solldosis an den jeweiligen Zielpunkten geschrieben. Viele der in dieser Anmeldung verwendeten Begriffe, beispielsweise Zielvolumen, Zielpunkt, Solldosis, Dosisverteilung etc., sind im Übrigen in dem als Leitfaden akzeptierten ICRU Report 50 (mit dem Addendum Report 62) definiert.

Anschließend wird der Bestrahlungsplan in eine Anzahl von zeitlich konsekutiven Teilbestrahlungsplänen aufgeteilt. Das heißt, dass mit dem Bestrahlungsplan vorgegeben wird, die Teilbestrahlungspläne direkt aufeinanderfolgend einen nach dem anderen abzuarbeiten.

Die Zielpunkte des Zielvolumens werden in Teilmengen auf die Teilbestrahlungspläne aufgeteilt, wobei die Teilmengen über das gesamte Zielvolumen verteilt sind. Es werden zueinander benachbarte Zielpunkte zu jeweils unterschiedlichen Teilbestrahlungsplänen zugeordnet. Mit anderen Worten umfasst jeweils eine Teilmenge über das gesamte Zielvolumen verteilte und nicht direkt benachbarte Zielpunkte.

Die beiden erstgenannten Schritte entsprechen dem bekannten Verfahren zum Aufteilen eines Zielvolumens.

Hierbei werden bislang gemäß dem Stand der Technik typischerweise die Zielpunkte des Zielvolumens sukzessive, d.h. einer nach dem anderen, angefahren. Dieses Verfahren hat sich so etabliert, da es besonders einfach durchzuführen ist.

Die Zielpunkte können zu Isoenergieschichten zugeordnet sein, wodurch eine Isoenergieschicht mit konstanter Teilchenenergie, also insbesondere unveränderten Beschleunigereinstellungen, bestrahlt werden kann.

Im erfindungsgemäßen Verfahren wird der Bestrahlungsplan in zeitlich konsekutive Teilbestrahlungspläne aufgeteilt.

Zueinander benachbarte Zielpunkte des Zielvolumens werden zu unterschiedlichen Teilbestrahlungsplänen zugeordnet. Wenn die Teilbestrahlungspläne zeitlich konsekutiv abgearbeitet werden, so werden zueinander benachbarte Zielpunkte nicht zeitlich konsekutiv bestrahlt, sondern vielmehr der erste Zielpunkt in einem ersten Teilbestrahlungsplan, und die zu dem ersten Zielpunkt benachbarten Zielpunkte in zumindest einem weiteren Teilbestrahlungsplan. Dies kann die Robustheit der Dosisdeposition gegenüber Bewegungen des Zielvolumens erhöhen. Mit anderen Worten kann die Dosisdeposition an den jeweiligen Zielpunkten verbessert werden, indem zueinander benachbarte Zielpunkte nicht unmittelbar nacheinander sondern zeitlich versetzt angefahren werden. Durch die Aufteilung von zueinander benachbarten Zielpunkten zu unterschiedlichen Teilbestrahlungsplänen kann daher auch die Homogenität der Dosisverteilung erhöht werden. Gegebenenfalls kann es darüber hinaus mit diesem Verfahren ausreichen, einen Zielpunkt nur ein einziges Mal anzufahren, wodurch sich eine erhebliche Zeitersparnis realisieren lässt. Dies bedeutet, dass mit dem erfindungsgemäßen Verfahren unter Umständen gar kein Rescanning durchgeführt wird. Es wird aber nicht ausgeschlossen, dass das erfindungsgemäße Verfahren je nach der benötigten Zieldosisverteilung auch mit Rescanning kombiniert werden kann, um jeweils das bestmögliche Ergebnis zu erhalten.

Indem die Teilmengen der Zielpunkte der Teilbestrahlungspläne jeweils über das gesamte Zielvolumen verteilt sind, wird mit den Teilbestrahlungsplänen jeweils bereits das gesamte Zielvolumen erfasst. Mit anderen Worten wird mit dem ersten Teilbestrahlungsplan ein grobes Raster einer Dosisverteilung im Zielvolumen deponiert, und mit jedem folgenden Teilbestrahlungsplan wird die Homogenität der Dosisverteilung verfeinert, da jeweils Teilmengen an Zielpunkten angefahren werden, die zu den vorherigen Teilmengen benachbart sind. Insbesondere handelt es sich bei den Teilmengen an Zielpunkten um echte Teilmengen im mathematischen Sinne, d.h. um Untermengen der Gesamtmenge an Zielpunkten, die jeweils nicht die gesamte Anzahl an Zielpunkten umfassen.

Kommt es im späteren Verlauf bei der Applikation der Dosis an einem der Zielpunkte eines der Teilbestrahlungspläne beispielsweise zu einer unerwarteten oder falsch berechneten Bewegung des Zielvolumens, wodurch die zu applizierende Dosis nicht an dem Zielpunkt sondern fehlerhaft deponiert wird, so ist dies statistisch durch die vorgehenden oder folgenden Teilbestrahlungspläne zumindest teilweise ausgleichbar.

Bei jedem Einzelbestrahlungsvorgang, wobei ein Zielpunkt mit einer Einzeldosis belegt wird, wird der gesamte Eintrittkanal des Partikelstrahls mit einem als Vordosis bezeichneten Dosisbetrag belegt. In einem aufwändigen Bestrahlungsplanungsverfahren müssen all die entstehenden Vordosen berücksichtigt und zur Berechnung der Gesamtdosis herangezogen werden. So ist es beispielsweise vorteilhaft, zunächst das distale Ende eines Zielvolumens zu bestrahlen, wobei der proximalere Teil des Zielvolumens bereits mit einer Vordosis belastet wird. Anschließend kann das Zielvolumen beispielsweise aus der Gegenrichtung bestrahlt werden, so dass der von dieser Seite aus distale Teil des Zielvolumens eine weitere Teildosis erhält. Im Idealfall entspricht die Dosisverteilung über das Zielvolumen einer Stufenfunktion. Dies ist naturgemäß im Praxisfall nur schwer zu erreichen.

Die Zielpunkte können den Teilmengen örtlich alternierend zugeordnet sein. Dies bedeutet, dass ein erster Zielpunkt der ersten Teilmenge zugeordnet ist, ein zweiter Zielpunkt der zweiten Teilmenge zugeordnet sein kann. Insbesondere sind die Zielpunkte den Teilmengen in den beiden Richtungen quer zur Strahlachse, also lateral, und/oder in drei Dimensionen örtlich alternierend den Teilmengen zugeordnet. Die Anzahl der Teilbestrahlungspläne bestimmt hierbei auch die Anzahl an Teilmengen der Zielpunkte.

Die Zielpunkte der Teilmengen sind daher bevorzugt nur von solchen Zielpunkten benachbart umgeben, die den jeweils anderen Teilmengen zugeordnet sind. Die Zielpunkte können bevorzugt zeilen- und/oder spaltenweise schachbrettartig alternierend den unterschiedlichen Teilbestrahlungsplänen zugeordnet sein. Insbesondere können in einer ersten Zeile oder Spalte Zielpunkte der ersten und zweiten Teilmenge und in einer zweiten Zeile oder Spalte Zielpunkte der dritten und vierten Teilmenge jeweils schachbrettartig angeordnet sein. In diesem Fall sind für den schachbrettartigen Aufbau nur zwei "Farben" des Schachbrettmustern zu betrachten, unter der Prämisse, dass die zu den Farben zugeordneten Teilmengen in jeweils geraden bzw. ungeraden Zeilen oder Spalten alterniert.

Die Anzahl der Teilbestrahlungspläne kann in dieser Analogiebetrachtung der Anzahl "Farben" des Schachbretts entsprechen, und dies kann ein mehrfarbiges Schachbrettmuster bedeuten, insbesondere sind die Zielpunkte dann im dreidimensionalen Fall schachbrettartig alternierend den unterschiedlichen Teilbestrahlungsplänen zugeordnet, wobei ein Feld (in der Analogie des Schachbretts) stets von Feldern anderer Farben benachbart ist.

Beim Anfahren der Zielpunkte der Teilbestrahlungspläne mit dem gescannten Strahl der Partikelanlage werden bevorzugt innerhalb der Teilbestrahlungspläne benachbarte Zielpunkte des Zielvolumens übersprungen. Dies bedeutet, dass der erste Zielpunkt angefahren wird, der örtlich dazu benachbarte Zielpunkt des Zielvolumens dagegen übersprungen wird. Der dritte Zielpunkt der Isoenergieschicht bzw. des Zielvolumens kann dann wieder ein Zielpunkt des ersten Teilbestrahlungsplans sein und somit angefahren werden. Insbesondere werden beim Anfahren der Zielpunkte jedes der Teilbestrahlungspläne benachbarte Zielpunkte des Zielvolumens übersprungen. Mit anderen Worten werden die Zielpunkte der Teilbestrahlungspläne derart angefahren, dass benachbarte Zielpunkte des Zielvolumens nicht unmittelbar aufeinanderfolgen.

Die Zielpunkte von zumindest zwei Teilmengen können mäanderförmig miteinander verschränkt im Zielvolumen angefahren werden.

Insbesondere überschneiden sich die Teilmengen der Zielpunkte nicht untereinander. Mit anderen Worten werden die Zielpunkte jeweils nur ein einziges Mal angefahren.

In einer bevorzugten Ausführungsform der Erfindung erstreckt sich ein Zielgebiet des Zielvolumens über mehrere Zielpunkte des Zielgebiets, wobei das Zielgebiet dem erwarteten Strahldurchmesser des gescannten Strahls entspricht. Die Zielpunkte in einem Zielgebiet werden als Gruppe von Zielpunkten, oder Gruppe, bezeichnet. Mit anderen Worten überdeckt ein Schuss des gescannten Strahls mehrere Zielpunkte, so dass an den Zielpunkten des Zielgebiets von einem Schuss des gescannten Strahls jeweils eine Dosis deponiert werden kann. Die Zielpunkte der Zielgebiete werden bevorzugt jeweils zu unterschiedlichen Teilmengen zugeordnet, so dass die Zielpunkte gleichermaßen das Zielgebiet auf die verschiedenen Teilbestrahlungspläne aufteilen. Daran, dass ein Zielgebiet von mehreren Teilbestrahlungsplänen erfasst wird, ist vorteilhaft, dass trotz einer Bewegung des Zielvolumens das jeweilige Zielgebiet zumindest mit einem Teil der Teilbestrahlungspläne angefahren werden kann. Dies sorgt für eine gewisse statistische Mittelung und somit für eine gleichmäßigere Dosisverteilung in einem bewegten Zielvolumen.

Die Anzahl der Zielpunkte in einem Zielgebiet kann besonders vorteilhaft der Anzahl der Teilbestrahlungspläne entsprechen. Mit anderen Worten ergibt sich die Anzahl der Teilbestrahlungspläne daraus, wie viele Zielpunkte in einem Zielgebiet liegen. Die Zielpunkte des Zielgebiets werden hierbei unterschiedlichen Teilmengen zugeordnet. Idealerweise wird jedes Zielgebiet dabei von jedem Teilbestrahlungsplan beschickt. Mit anderen Worten wird von den in einem gemeinsamen Zielgebiet angeordneten Zielpunkten höchstens jeweils ein Zielpunkt von jedem Teilbestrahlungsplan angefahren. Es soll aber dabei nicht ausgeschlossen sein, dass in komplexen Zielvolumenformen, die ggf. eng von Risikobereichen (OAR, Organs at Risk) umgeben sind, Teilbestrahlungspläne vorteilhaft sind, in denen jeweils zumindest ein Zielpunkt eines Zielgebiets angefahren wird, wobei diese Teilbestrahlungspläne echte Untermengen der Menge an Zielpunkten im Zielvolumen umfassen.

Werden die Zielpunkte zu Isoenergieschichten zusammengefasst und befinden sich Teile der Zielpunkte der Teilmengen in einer Isoenergieschicht, so ist es vorteilhaft, diese Teile der Zielpunkte konsekutiv anzufahren. Mit anderen Worten umfasst jede Isoenergieschicht Zielpunkte von mehreren oder allen Teilmengen. Hierdurch können die Teile der Zielpunkte des Bestrahlungsplans, die in einer Isoenergieschicht liegen, ggf. ohne Änderung der Beschleunigereinstellungen angefahren werden.

Beispielsweise können die zu einer Isoenergieschicht zusammengefassten Teilmengen an Zielpunkten jeweils abwechselnd zeilenweise und spaltenweise angefahren werden. Mit anderen Worten werden die Zielpunkte von zumindest zwei der Teilmengen in den Isoenergieschichten abwechselnd zeilenweise und spaltenweise angefahren. Hierbei ist es ein besonders vorteilhaftes Merkmal des Verfahrens, dass beispielsweise jede zweite Zeile oder jede zweite Spalte der Zielpunkte einer Isoenergieschicht übersprungen werden kann. Dies soll nicht ausschließen, dass es mit dem erfindungsgemäßen Verfahren auch möglich ist, beispielsweise jede vierte oder sechste Zeile oder Spalte der Zielpunkte einer Isoenergieschicht zu überspringen, jedoch ist es besonders vorteilhaft, jede zweite Zeile oder Spalte der Zielpunkte einer Isoenergieschicht zu überspringen.

Besonders vorteilhaft weist der Bestrahlungsplan zumindest vier Teilbestrahlungspläne auf. Ein Zielgebiet umfasst in diesem Fall demnach zumindest vier Zielpunkte, die zu unterschiedlichen Teilbestrahlungsplänen zugeordnet werden. Der Abstand der Isoenergieschichten zueinander, also der Abstand der Zielpunkte in Strahlrichtung, ist dabei bevorzugt so gewählt, dass die Zielpunkte eines Zielgebiets in einer Isoenergieschicht angeordnet sind. Mit anderen Worten werden Zielpunkte benachbarter Isoenergieschichten beim Anfahren der Zielpunkte der Isoenergieschicht nur gering beeinflusst.

Die Anzahl der Zielpunkte pro Zielgebiet lässt sich anpassen, indem entweder der Strahldurchmesser variiert oder der Punktabstand der Zielpunkte von einander variiert wird. Je nach der zu erwartenden Bewegung kann daher ein Zielgebiet eine unterschiedliche Anzahl an Zielpunkten aufweisen. Daher soll nicht ausgeschlossen sein, dass manche Zielgebiete von manchen Teilbestrahlungsplänen nicht angefahren werden, da sich das Zielgebiet aufgrund des Rasters keine genügend große Anzahl an Zielpunkten aufweist. Die Zielpunkte der Zielgebiete werden dann einfach auf möglichst viele Teilbestrahlungspläne aufgeteilt, so dass auch hier bereits der vorteilhafte Effekt der statistischen Mittelung auftritt. Es ist auch eine Möglichkeit, Zielpunkte gleichzeitig in verschiedene, sich dann teilweise überdeckende Teilmengen aufzunehmen, so dass diese Zielpunkte mehrfach angefahren werden und die Zielgebiete mit jedem Teilbestrahlungsplan erfasst werden.

Bei einer Variation des Rasterabstands kann auch die Vordosis berücksichtigt werden, indem der Punktabstand über das Zielvolumen variiert wird, also keine konstante Größe über das Zielvolumen darstellt. Hierdurch lässt sich das Raster derart anpassen, dass ggf. doch jedes Zielgebiet von jedem Teilbestrahlungsplan erfasst wird, ohne dass Zielpunkte mehrfach angefahren werden.

Bei der Festlegung der Anzahl von Teilbestrahlungsplänen kann ferner die Anzahl der Zielgebiete, die lokalen Bewegungsparameter und/oder die Vordosis, die beim Anfahren von anderen Zielpunkten entsteht, berücksichtigt werden. So kann bei einer gleichförmigen Bewegung, bei der keine Fluktuationen zu erwarten sind, eine kleine Anzahl Teilbestrahlungspläne ausreichend sein. Bei einer komplexen Bewegung, die schwer vorherzusehen ist, kann es sinnvoll sein, eine größere Anzahl Teilbestrahlungspläne festzulegen. Die größere Anzahl Teilbestrahlungspläne kann hierbei durch ein feineres Punktraster, also durch einen geringeren Punktabstand der Zielpunkte zueinander erreicht werden.

Im Rahmen der Erfindung wird auch eine Steuerungsvorrichtung bereitgestellt, die die Schritte der oben genannten Verfahren ausführen kann.

Im Folgenden wird die Erfindung anhand von Ausführungsbeispielen und unter Bezugnahme auf die Figuren näher erläutert, wobei gleiche und ähnliche Elemente teilweise mit gleichen Bezugszeichen versehen sind und die Merkmale der verschiedenen Ausführungsbeispiele miteinander kombiniert werden können.

### Kurzbeschreibung der Figuren

Es zeigen:
- Fig. 1: eine Übersicht über den Aufbau einer typischen Bestrahlungsanlage
- Fig. 2: eine schematische Darstellung von zur Steuerung einer Bestrahlungsanlage verwendeten Komponenten
- Fig. 3: eine schematische Darstellung einer Ablenk- und Modulationseinrichtung sowie eine Bestrahlung mit aktiver Bewegungskompensation
- Fig. 4: den Gesamtbestrahlungsplan einer Isoenergieschicht
- Fig. 5: den Gesamtbestrahlungsplan einer Isoenergieschicht, wobei die Zielpunkte unterteilt sind in A, B, C und D,
- Fig. 6: einen Teilbestrahlungsplan als Untermenge des Gesamtbestrahlungsplans gemäß Fig. 4 umfassend die Punkte A,
- Fig. 7: den Teilbestrahlungsplan umfassend die Punkte B,
- Fig. 8: den Teilbestrahlungsplan umfassend die Punkte C,
- Fig. 9: den Teilbestrahlungsplan umfassend die Punkte D,
- Fig. 10: eine Übersicht über ausführbare Schritte des Bestrahlungsplanungsverfahrens in den Varianten der Fig. 10a, Fig. 10b und Fig. 10c.

### Detaillierte Beschreibung der Figuren

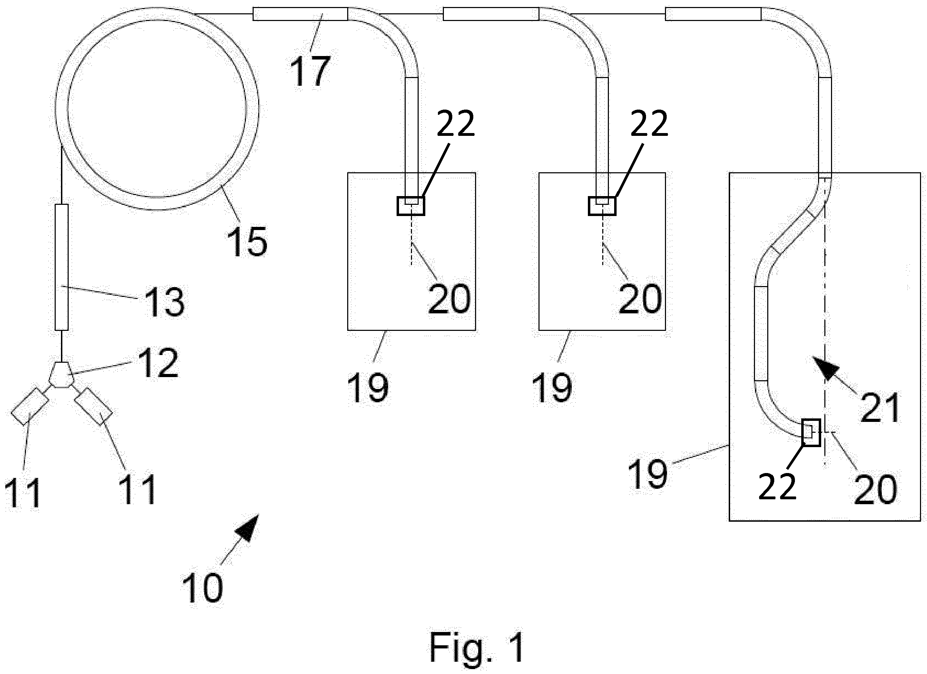

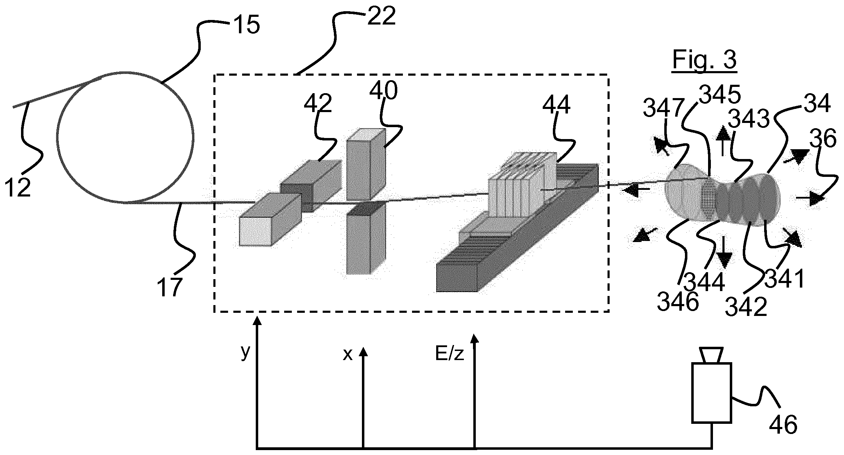

Figur 1 zeigt den schematischen Aufbau einer an sich bekannten Partikeltherapieanlage 10. Die Partikeltherapieanlage 10 erzeugt und beschleunigt geladene Partikel, die in Form eines Partikelstrahls 20 zur weiteren Verwendung bereitgestellt werden und mittels einer Strahlführung 17 in ein definierbares Zielvolumen 34 (siehe Figur 3) lenkbar sind. Das Zielvolumen 34 enthält beispielsweise im Rahmen einer Tumortherapie einen Tumor, es kann aber auch zu wissenschaftlichen Zwecken, Tierversuchen, Modell- und Materialproben und Allgemein zur Erforschung des Partikelstrahls und/oder der Partikeltherapie ein Zielvolumen 34 definiert sein, welches unbelebtes Material und/oder Zellkulturen enthält. Die Partikeltherapieanlage 10 wird auch zur Bestrahlung von Phantomen mit dem Partikelstrahl 20 eingesetzt, mittels welchen eine Vielzahl an Bestrahlungsparametern vor und/oder nach einer erfolgten Bestrahlung bzw. Behandlung eines Patienten verifiziert werden kann.

Die Partikel werden in dem in Figur 1 gezeigten Beispiel in einer der zwei Ionenquellen 11 erzeugt und vorbeschleunigt. Die Ionenquellen 11 können insbesondere verschiedenste Teilchen von Protonen bis Uran erzeugen, aufgrund ihrer für die Partikeltherapie vorteilhaften Eigenschaften wie der charakteristischen (teilchenabhängigen) Wechselwirkung mit Materie und der Eindringtiefe werden bevorzugt Protonen, Pionen, Heliumionen oder besonders bevorzugt Kohlenstoffionen eingesetzt. Ganz allgemein werden bevorzugt Hadronen als Partikel eingesetzt. Mittels einer Niedrigenergiestrahlführung 12 werden die Partikel in einen Vorbeschleuniger 13, im gezeigten Fall ein Linearbeschleuniger 13, eingefädelt. Der Linearbeschleuniger 13 beschleunigt die Partikel auf eine erste Energiestufe und bündelt die Partikel zu einem Partikelstrahl 20. Mit der ersten Energiestufe werden die Partikel schließlich in die Beschleunigereinheit 15, z.B. wie dargestellt ein Synchrotron, aber auch ein Zyklotron, mit einer weiteren Niedrigenergiestrahlführung 12 eingefädelt und dort weiter bis auf die für die jeweilige Anwendung einstellbare Extraktionsenergie beschleunigt. Die Strahlführung 17 führt den Partikelstrahl 20 schließlich an das gewünschte Ziel, genannt "Target", in einem Messraum 19 oder Therapieraum 21, wo der Partikelstrahl 20 mit einem typischen Strahldurchmesser von 3 bis 30 Millimetern angewendet werden kann bzw. dort bereitgestellt wird.

Zur genauen Positionierung des Partikelstrahls 20 zum Anfahren eines Zielpunkts 30 eines Zielvolumens 34 in einem Körper 77 befindet sich im Mess- oder Bestrahlungsraum 19 oder Therapieraum 21 eine Ablenk- und Modulationseinrichtung 22 zum lateralen, also horizontalen und vertikalen, Ablenken des Partikelstrahls 20 und zur Energiemodulation zum schnellen Variieren der Partikelstrahlenergie, die die Eindringtiefe des Partikelstrahls 20 bestimmt. Da hiermit ein ganzes Raster an Zielpunkten in einem Zielvolumen sukzessive angefahren werden kann und das sukzessive Anfahren der Zielpunkte als "Scannen" bezeichnet wird, wird die Einrichtung als Rasterscaneinrichtung 22 bezeichnet.

Ganz allgemein können die Bestrahlungsverfahren Spotscan, kontinuierliche Bestrahlung und Rasterscan eingesetzt werden.

Die Reihenfolge des Anfahrens der Zielpunkte 30 des Zielvolumens 34 wird in einem Bestrahlungsplan festgehalten, der ferner weitere wesentliche Parameter enthalten kann, wie insbesondere die Parameter des Zielvolumens 34 und/oder eine zu erwartende Bewegung des Zielvolumens 34. Einer der wesentlichen Vorzüge der Rasterscaneinrichtung 22 ist, dass diese die Möglichkeit bereitstellt, den Partikelstrahl 20 kontinuierlich auf das Zielvolumen 34 zu richten.

Die gesamte Partikeltherapieanlage 10 wird schließlich von einem Beschleunigerkontrollsystem gesteuert, welches insbesondere die Beschleunigereinheit 15 sowie die Strahlführung 17 steuert und Messdaten zur Überwachung von Strahlparametern sammelt. Gegebenenfalls können die Parameter zur Steuerung der Partikeltherapieanlage 10 basierend auf dem Bestrahlungsplan eingestellt werden, so dass der Bestrahlungsplan auch die Einstelldaten zur Steuerung der Partikeltherapieanlage 10 umfasst.

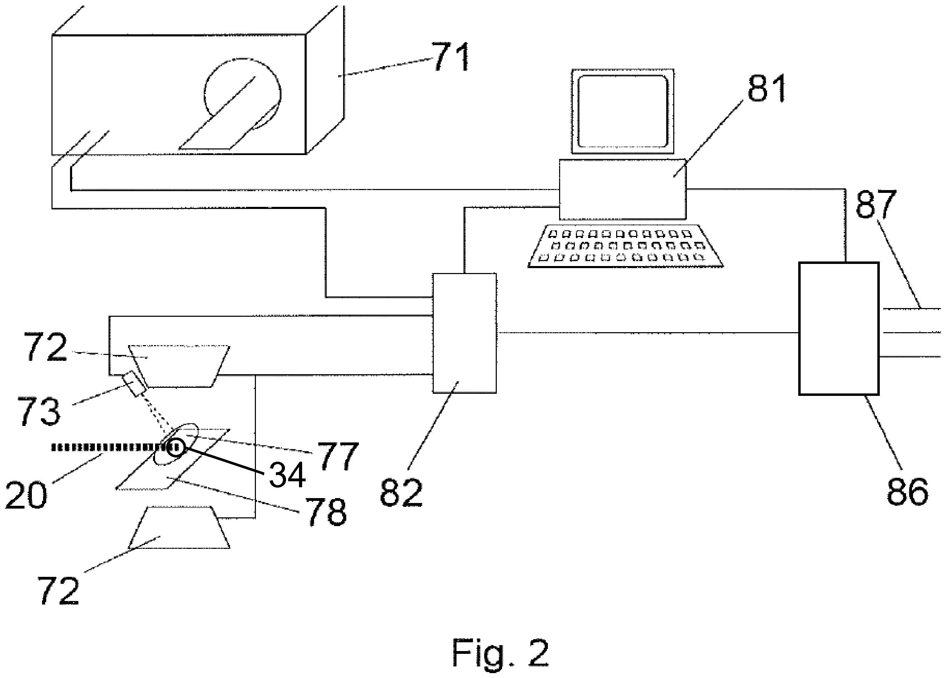

Figur 2 zeigt eine schematische Darstellung von an sich bekannten Einrichtungen, die bei der Erstellung eines Bestrahlungsplanes, also der Bestrahlungsplanung, zum Erzeugen eines Datensatzes, der Zielpunkte 30 in einem Zielvolumen 34 in einem Körper 77 definiert, und bei der Steuerung einer Bestrahlungsanlage 10, wie sie beispielsweise anhand der Figur 1 dargestellt wurde, verwendet werden können.

Mittels eines Computer-Tomographen oder Kernspin-Tomographen 71 oder mittels anderer diagnostischer Einrichtungen können Lage und Ausdehnung eines zu bestrahlenden Tumors oder eines anderen Zielvolumens 34 ermittelt werden. Daten von dem Tomographen 71 werden unmittelbar oder nach einer Aufbereitung durch weitere, in Figur 2 nicht dargestellte Einrichtungen in einer Vorrichtung 81 zum Erstellen eines Datensatzes verarbeitet. Die Vorrichtung 81 ist beispielsweise ein Arbeitsplatz-Computer, eine Workstation oder ein anderer Computer. Optional ist die Vorrichtung 81 ferner durch ihre Benutzerschnittstelle, Software oder andere Merkmale dafür geeignet, dass medizinisches Personal dort das Zielvolumen 34, die zu applizierenden Dosen, die Aufteilung derselben auf mehrere Fraktionen, die Richtung der Bestrahlung und andere Details der Partikeltherapie definiert.

Der zu bestrahlende Körper 77 kann mit verschieden ausgebildeten Überwachungseinrichtungen vor, während oder nach der Bestrahlung durch die Partikeltherapieanlage 10 überwacht werden. Beispielsweise sind eine PET-Kamera 72 (PET = Positronen-Emissions-Tomographie) und/oder ein Abstandssensor 73 zur Erfassung eines zu bestrahlenden Körpers 77, der auf einer Lagerfläche 78 gelagert ist, vorgesehen. Die PET-Kamera 72 und/oder der Abstandssensor 73 und die Lagerfläche 78 können innerhalb eines der oben anhand der Figur 1 dargestellten Bestrahlungsräume 19 angeordnet sein. In diesem Fall können mittels der PET-Kamera 72 und/oder des Abstandssensor 73 die durch einen Partikelstrahl 20 erzeugte Dosis sowie Bewegungen des bestrahlten Körpers 77 erfasst werden. Alternativ sind die PET-Kamera 72, der Abstandssensor 73 und die Lagerfläche 78 außerhalb eines Bestrahlungsraums angeordnet. Alternativ oder zusätzlich kann der Körper 77 mittels einer Fluoroskopie-Einrichtung, einer Röntgen-Einrichtung, eines Ultraschallsensors, eines Atemgürtels und/oder anderer externer Sensoren überwacht werden.

Daten vom Tomographen 71, von der PET-Kamera 72 und vom Abstandssensor 73 können von einer Einrichtung 82 zum Bestimmen von einem oder mehreren Bewegungsparametern verarbeitet werden. Mittels der Einrichtung 82 können vor einer Bestrahlung oder während einer Bestrahlung Bewegungen von Teilbereichen des Körpers 77 (beispielsweise aufgrund von Atmung oder Herzschlag) quantitativ erfasst werden. Der oder die von der Einrichtung 82 bestimmten Bewegungsparameter können von der Vorrichtung 81 zum Erstellen eines Datensatzes berücksichtigt werden.

Zur Berücksichtigung bei der Erstellung eines Datensatzes eignen sich insbesondere Daten über die Amplituden typischer und/oder periodischer Bewegungen oder über einen Zusammenhang zwischen der räumlichen Lage des Zielvolumens und/oder einer von außen, beispielsweise mittels des Abstandssensors 73 erfassbaren Größe. Alternativ oder zusätzlich können von der Einrichtung 82 bestimmte Parameter bzw. Daten direkt von einer Steuerung 86 zur Steuerung einer Bestrahlungsanlage 10, wie sie anhand der Figur 1 dargestellt wurde, verarbeitet werden. Dazu eignen sich besonders Daten, die während der Bestrahlung von der PET-Kamera 72 oder dem Abstandssensor 73 erfasst werden. In die Steuerung der Anlage 10 durch die Steuerungseinheit 86 geht ferner der von der Vorrichtung 81 erstellte Datensatz ein. Über Steuerleitungen 87 oder auf andere Weise ist die Steuerungseinheit 86 mit der Bestrahlungsanlage 10 gekoppelt.

Der anhand der Figur 1 dargestellte Grundaufbau einer Bestrahlungsanlage 10 ist typisch für viele Partikeltherapieanlagen und andere Bestrahlungsanlagen. Die nachfolgend beschriebenen Ausführungsbeispiele sind sowohl in Zusammenhang mit der anhand der Figur 1 dargestellten Bestrahlungsanlage und den anhand der Figur 2 dargestellten Einrichtungen als auch mit anderen Bestrahlungsanlagen und Einrichtungen einsetzbar.

Figur 3 zeigt schematisch eine Bestrahlung mit aktiver Bewegungskompensation. Die Beschleunigereinheit 15 stellt den Partikelstrahl 20 bereit, welcher mit zwei Magnetscanpaaren 40, 42 lateral über das Zielvolumen 34 gerastert wird. Die Zielpunkte 30 des Zielvolumens 34 definieren das Punktraster des Zielvolumens, wobei die Zielpunkte in einer Vielzahl an Isoenergieschichten, im gezeigten Beispiel die Isoenergieschichten 341 bis 347, angeordnet sind. Die Isoenergieschichten 341 bis 347 werden sukzessive mit dem Partikelstrahl 20 abgerastert. In der Ausführungsform der Figur 3 wird gerade die Isoenergieschicht 345 lateral abgerastert. Bewegt sich das Zielvolumen 34 wie durch die Pfeile 36 symbolisiert ist, wird die Strahlposition bei Kenntnis der Bewegung der aktuell bestrahlten Rasterposition i lateral mittels der Scannermagnete 40, 42 und longitudinal mittels eines Doppelkeilsystems 44 aktiv der Bewegung des Zielvolumens 34 nachgeführt, um trotz Bewegung des Zielvolumens 34 die beabsichtigte Rasterposition i zu treffen. Mit anderen Worten wird der Partikelstrahl 20 mittels der Ablenk- und Modulationseinrichtung 22, insbesondere umfassend die Scannermagnete 40, 42 und das Doppelkeilsystem 44, der Bewegung nachgeführt. Die Istposition der gerade bestrahlten Rasterposition i wird mittels einer Bewegungserfassungseinrichtung 46 bestimmt, die die Bewegung des Körpers 77 erfasst und eine Positionsadaptionstabelle erzeugt.

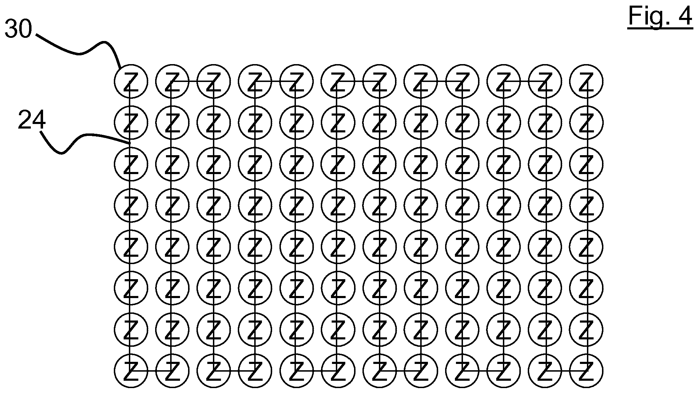

Figur 4 zeigt eine Ausführungsform eines an sich bekannten Bestrahlungsplans, wobei bei den Figuren 4 bis 9 aus Anschaulichkeitsgründen jeweils nur eine Isoenergieschicht des Zielvolumens 34, und zwar in dem Beispiel die aus Figur 3 bekannte Isoenergieschicht 345, dargestellt ist. Die in Figur 4 die Zielpunkte 30 miteinander verbindende Linie symbolisiert den Scanpfad 24 des gescannten Partikelstrahls 20, wie dieser in einem an sich bekannten Ablauf jeweils zueinander benachbarte Zielpunkte 30 sukzessive anfährt. Mit anderen Worten werden die Zielpunkte 30 der gezeigten Isoenergieschicht 345 Nachbarpunkt für Nachbarpunkt mit dem gescannten Partikelstrahl 20 abgerastert. Demnach werden die in Figur 4 gezeigten jeweils zueinander benachbarten Zielpunkte 30 sukzessiv angefahren.

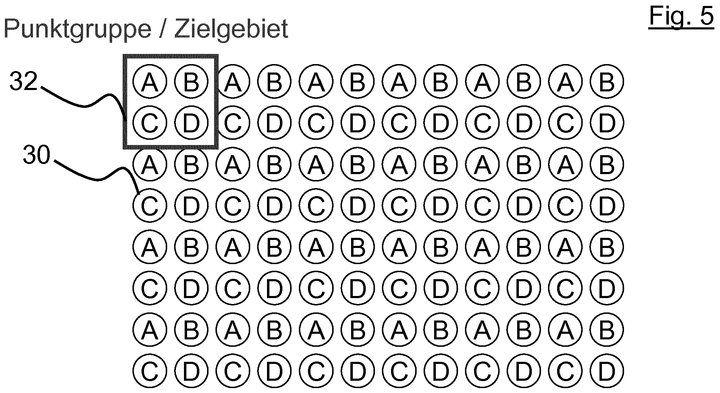

Figur 5 zeigt eine Ausführungsform eines Bestrahlungsplans, bei dem die Zielpunkte 30 eines jeden Zielgebiets 32, welches symbolisch durch ein Quadrat gekennzeichnet ist, in die Teilmengen A, B, C und D aufgeteilt sind. Die Größe der Zielgebiete 32 ergibt sich aus dem Strahldurchmesser des eingesetzten Partikelstrahls 20. In der gezeigten Ausführungsform entspricht der Abstand von einer Ecke des Symbolquadrats zur nächsten Ecke dem Durchmesser des typischerweise kreisrunden Strahldurchmessers. Die Teilmenge A ist dem Teilbestrahlungsplan A zugeordnet, die Teilmengen B, C und D sind den jeweiligen Teilbestrahlungsplänen B, C und D zugeordnet. Die Teilbestrahlungspläne A, B, C und D werden zeitlich konsekutiv abgearbeitet. Mit anderen Worten wird zuerst die Teilmenge A an Zielpunkten 30 des Teilbestrahlungsplans A angefahren, daran anschließend die Teilmenge B an Zielpunkten 30 des Teilbestrahlungsplans B, die Teilmenge C an Zielpunkten 30 des Teilbestrahlungsplans C und schließlich die Teilmenge D an Zielpunkten 30 des Teilbestrahlungsplans D.

Der Partikelstrahl 20 deponiert beim Anfahren eines Zielpunktes 30 bevorzugt im gesamten Zielgebiet 32 eine Dosisverteilung. Die Dosisverteilung ist dabei typischerweise abhängig von Profil und Durchmesser des eingesetzten Partikelstrahls, insbesondere weist der Partikelstrahl zumeist einen runden Querschnitt auf, so dass auch die Dosisverteilung konzentrisch vom Strahlmittelpunkt aus betrachtet nach außen hin abnimmt. Das Zielgebiet 32 zeigt daher nur eine für das einfache Verständnis dienende Darstellung des Zusammenhangs zwischen Dosisdeposition und davon betroffenen Zielpunkten 30.

Die Darstellung nur einer Isoenergieschicht des Zielvolumens, wie in den Figuren 4 bis 9 gezeigt, ermöglicht einen besonders einfachen Zugang zu den Besonderheiten des erfindungsgemäßen Verfahrens. Für den Fachmann ist es nicht notwendig, eine dreidimensionale Dosisdeposition, die ggf. auch Zielpunkte 30 benachbarter Isoenergieschichten 344, 346 betrifft, die Berechnung der dreidimensionalen Vordosen und die Auswahl der Bestrahlungsreihenfolge der Isoenergieschichten im Detail zu klären, da dieser vielmehr bereits daraus und aus den Figuren auf den vollständigen Bestrahlungsvorgang rückschließen kann und insbesondere den Bestrahlungsplan für alle Isoenergieschichten 341 bis 347 gleichermaßen anpassen. So kann das Verfahren anhand der Beschreibung und der Figuren leicht auf den dreidimensionalen Fall mit einer Mehrzahl an Isoenergieschichten 341 bis 347 und/oder mit räumlich im Zielvolumen 34 verteilten Zielpunkten 30 übertragen werden.

Insbesondere können auch die zu anderen Isoenergieschichten 341 bis 344 und 346 bis 347 zugeordneten Zielpunkte 30, die zu dem Zielpunkt der betrachteten Isoenergieschicht 345 benachbart sind, zu anderen Teilmengen zugeordnet sein, so dass auch eine in Strahlrichtung räumliche Benachbarung von Zielpunkten 30 bei der Aufteilung der Teilmengen von Zielpunkten 30 berücksichtigt wird. Ggf. kann der Abstand der Isoenergieschichten 341 bis 347 zueinander auch so gewählt sein, dass sich die dreidimensionale Dosisverteilung in einem Zielgebiet 32 gerade nicht auf die Teilmengen A, B, C, D der benachbarten Isoenergieschichten 344 und/oder 346 auswirkt, sondern die benachbarten Isoenergieschichten eine ausreichend großen Abstand zu der Isoenergieschicht 345 aufweisen.

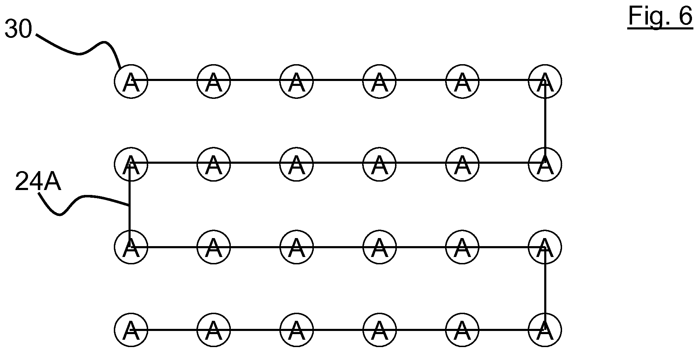

Fig. 6 zeigt die Teilmenge A der bereits mit Fig. 4 gezeigten Menge an Zielpunkten 30, wobei die Zielpunkte 30 der Teilmenge A zeitlich konsekutiv angefahren werden. Das heißt, dass die Teilmenge A der Zielpunkte 30 in einem ersten Teilbestrahlungsplan A angefahren wird. In dem vereinfachten Beispiel der Fig. 6 der gezeigten Isoenergieschicht 345 bildet die Teilmenge A den ersten Teilbestrahlungsplan vollständig ab. Die Zielpunkte 30 der Teilmengen an Zielpunkten B, C und D, die zu den weiteren Teilbestrahlungsplänen B, C und D zugeordnet sind, werden übersprungen. In dem gezeigten Beispiel der Fig. 6 werden die Zielpunkte 30 zeilenweise angefahren, wie der Scanpfad 24A des gescannten Partikelstrahls 20 symbolisiert.

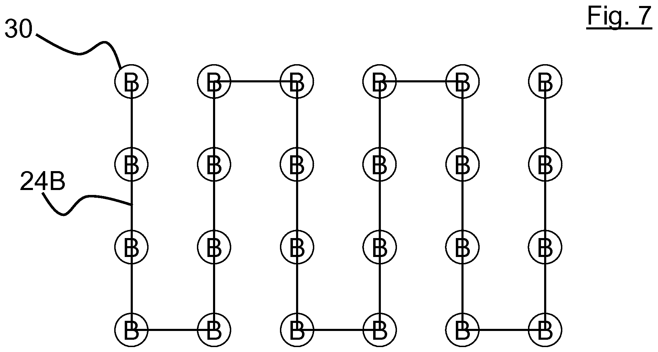

Figur 7 zeigt die Teilmenge B der mit Fig. 4 gezeigten Zielpunkte 30, wobei die Teilmenge B mit dem Teilbestrahlungsplan B sukzessive angefahren wird. Jeder der Zielpunkte 30 der Teilmenge B ist zu einem der Zielpunkte 30 der Teilmenge A benachbart und nicht zu demselben Teilbestrahlungsplan zugeordnet. In dem gezeigten Beispiel befinden sich die Zielpunkte 30 der Teilmenge B in der gleichen Isoenergieschicht 345 wie diejenigen der Teilmenge A. Die Zielpunkte 30 der Teilmenge B können, wie gezeigt, spaltenweise angefahren werden, wie durch den Scanpfad 24B des gescannten Partikelstrahls 20 symbolisiert wird. Wird in einem Teilbestrahlungsplan B die Teilmenge B der Zielpunkte 30 spaltenweise und in einem davor und/oder danach abzuarbeitenden weiteren Teilbestrahlungsplan A die Teilmenge A der Zielpunkte 30 zeilenweise angefahren, ergibt sich durch das sukzessive Abarbeiten der Teilbestrahlungspläne A und B ein Überkreuzraster der Dosisdeposition im Zielvolumen 34, insbesondere in der Isoenergieschicht 345. Gegebenenfalls kann das Anfahren des ersten Zielpunktes 30 des Teilbestrahlungsplans B mit dem zu dem ersten Zielpunkt 30 des Teilbestrahlungsplans A benachbarten Zielpunkt 30 begonnen werden, ein Beginn an einem anderen Bereich der Isoenergieschicht 345 ist dabei ebenfalls denkbar. Insbesondere dann, wenn das Zielvolumen aus unterschiedlichen Richtungen, beispielsweise aus gegenüberliegenden Richtungen, bestrahlt werden soll kann es vorteilhaft sein, die Teilbestrahlungspläne an verschiedenen Orten im Zielvolumen 34 bzw. in der Isoenergieschicht 345 beginnen zu lassen. Im gezeigten Fall nur einer Isoenergieschicht 345 bietet sich das Überkreuzraster aufeinanderfolgender Teilbestrahlungspläne aufgrund der Vermeidung von Resonanzeffekten im Falle von bewegten Zielvolumen 34 an.

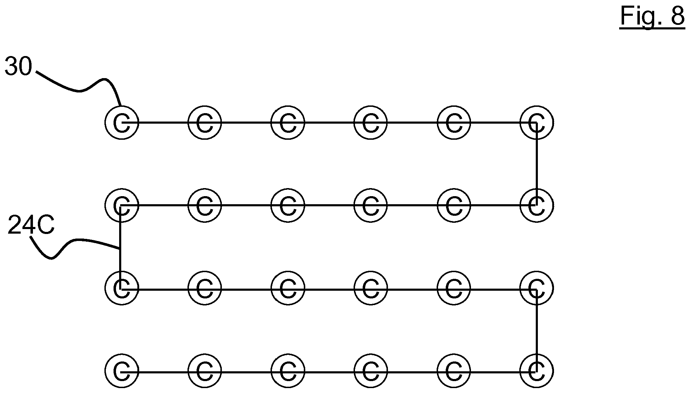

Figur 8 zeigt den Teilbestrahlungsplan C, dessen Zielpunkte 30 einer Teilmenge der in Fig. 4 gezeigten Zielpunkte 30, nämlich den mit "C" markierten, entspricht. Analog des in Fig. 5 gezeigten Anfahrens der Zielpunkte A werden auch die auf den Teilbestrahlungsplan B folgenden Zielpunkte 30 zeilenweise bestrahlt, wie durch den Scanpfad 24C des gescannten Partikelstrahls 20 symbolisiert wird. Demgemäß werden die Zielpunkte 30 abwechselnd zeilen- und spaltenweise angefahren. Dies kann Resonanzeffekte in der Dosisdepositionsverteilung verhindern.

Unter Resonanzeffekt wird beispielsweise verstanden, wenn die Regelmäßigkeit der Bewegung des Zielvolumens 34 mit dem Abarbeiten der Teilbestrahlungspläne in zeitliche Korrelation kommt. Zum Beispiel kann sich dies so manifestieren, dass die Zeitdauer, die zur Abarbeitung eines Teilbestrahlungsplans benötigt wird, dem Vielfachen der Amplitudendauer der Zielvolumenbewegung entspricht. Eine Fehldosierung an einem Zielpunkt 30 würde dann von jedem Teilbestrahlungsplan wiederholt werden, so dass der Fehlpunkt ggf. eine zu niedrige oder zu hohe Bestrahlungsdosis erhält. Indem also der Scanpfad 24A bis 24D, also das nacheinander Anfahren von Zielpunkten 30, von Teilbestrahlungsplan zu Teilbestrahlungsplan variiert wird, kann die Gleichmäßigkeit der Dosisdeposition im Zielvolumen 34 gesteigert werden. Bevorzugt können dadurch sogar Fehldosisapplikationen ausgeglichen bzw. verhindert werden, was die bisherigen Bestrahlungsverfahren in dieser Art nicht realisierten.

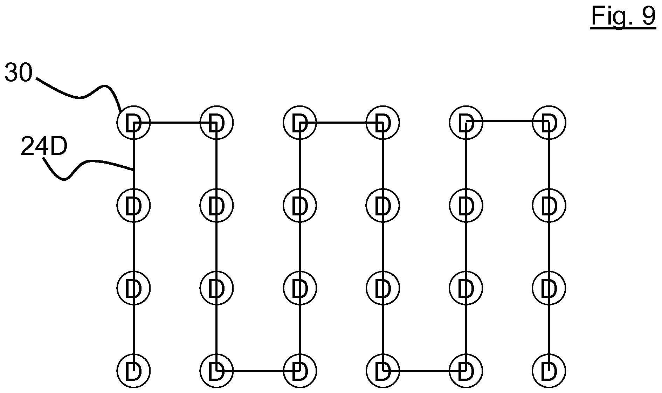

Figur 9 zeigt schließlich den Teilbestrahlungsplan, der die Teilmenge an Zielpunkten 30 umfasst, die mit dem Buchstaben "D" gekennzeichnet sind. Der Scanpfad 24D des gescannten Partikelstrahls zum Anfahren der Zielpunkte "D" erfolgt spaltenweise. Dieses spaltenweise Anfahren der Zielpunkte D entspricht dem Scanpfad 24B des mit Fig. 7 gezeigten Anfahrens der Zielpunkte B. Die Teilmengen B und D werden also spaltenweise gescannt, die Teilmengen A und C zeilenweise. Mit anderen Worten wechselt sich das zeilen- und spaltenweise Anfahren der Zielpunkte 30 unter den Teilbestrahlungsplänen ab, der Scanpfad 24A bis 24D des Partikelstrahls 20 alterniert zeilen- bzw. spaltenweise.

Figuren 9a, 9b und 9c zeigen in drei Beispielen das systematische Abarbeiten von Programmpunkten des Bestrahlungsplanes.

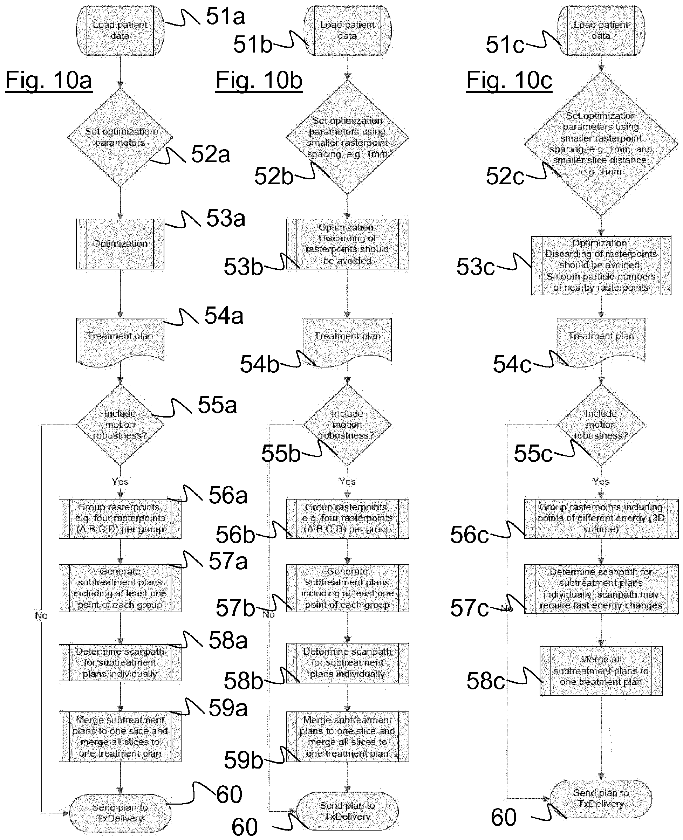

Figur 10a zeigt, dass in einem ersten Schritt 51 die Patientendaten aus dem Bestrahlungsplan geladen werden, welche auch die Lage und die Abmessungen des Zielvolumens 34 umfassen.

Unter Kenntnis der Patientendaten werden in einem zweiten Schritt 52a Optimierungsparameter in Abhängigkeit von den Patientendaten gesetzt, um zum Beispiel einen optimalen Scanpfad zu erhalten, der eine möglichst homogene Dosisverteilung und/oder eine möglichst kurze Bestrahlungsdauer realisiert. Hierbei können insbesondere die Zielpunkte 30 festgelegt werden.

In einem dritten Schritt 53a werden die Parameter angewendet und die Optimierungen durchgeführt, die schließlich in dem vierten Schritt 54a zu dem Vorergebnis eines einzelnen Bestrahlungsplans führen.

In dem fünften Schritt 55a kann von einem Benutzer vorgegeben werden, ob eine Bewegung des Zielvolumens 30 zu erwarten ist und demgemäß eine Verbesserung der Dosisverteilungshomogenität berechnet werden soll.

Eine verbesserte Berücksichtigung der Zielvolumenbewegung führt zu dem sechsten Schritt 56a, in welchem die Zielpunkte 30 zu den Teilmengen, beispielsweise A, B, C und D, zugeordnet werden.

Mit anderen Worten werden die Zielpunkte 30 je Zielgebiet 32 gruppiert und aus jeder Gruppe ein Zielpunkt 30 zu einem Teilbestrahlungsplan zugeordnet. Insbesondere richtet sich die Gruppierung nach der Größe des Zielgebiets 32 und ein Zielgebiet 32 umfasst eine Gruppe an Zielpunkten 30.

In einem siebten Schritt 57a werden Teilbestrahlungspläne erzeugt und die Zielpunkte 30 den Teilbestrahlungsplänen zugeordnet. Jeder Teilbestrahlungsplan umfasst insbesondere mindestens einen Zielpunkt 30 jeder Gruppe.

Die einzelnen Scanpfade 24A bis 24D werden in einem achten Schritt 58a für jeden einzelnen Teilbestrahlungsplan festgelegt. Dies kann unabhängig voneinander erfolgen, es können aber auch bestimmte Regelmäßigkeiten der Scanpfade 24A bis 24D aufeinanderfolgender Teilbestrahlungspläne festgelegt werden, die beispielsweise zu einem Überkreuzraster des Anfahrens von Zielpunkten 30 führen.

Im neunten Schritt 59a werden die Teilbestrahlungspläne derart zusammengefasst, dass die Energie des Partikelstrahls 20 möglichst selten variiert werden muss, das heißt, es werden die Zielpunkte 30 einer Isoenergieschicht 341 bis 347 zusammengefasst und alle Teilbestrahlungspläne ergeben gemeinsam einen übergeordneten Bestrahlungsplan.

Der Bestrahlungsplan wird in einem zehnten Schritt 60 schließlich an das Beschleunigerkontrollsystem bzw. der Steuerung 86 zum Einstellen der Beschleunigerparameter übermittelt.

Fig. 10b zeigt ein weiteres Ablaufdiagramm, wobei in einem ersten Schritt 51b die Patientendaten geladen werden. In einem zweiten Schritt 52b werden die Optimierungsparameter gesetzt, wobei ein engerer Punktabstand der Zielpunkte 30 gesetzt wird, beispielsweise 1 mm. Hierbei wird in einem dritten Schritt 53b überprüft, dass möglichst wenige Zielpunkte 30 des Rasters der Zielpunkte im Zielvolumen 34 bei der Optimierung der Dosisverteilungsberechnung nicht angefahren werden. Mit anderen Worten soll die Dosis auf möglichst viele Zielpunkte 30 des Zielvolumens 34 verteilt werden. Die Optimierung führt schließlich in einem vierten Schritt 54b zu einem Bestrahlungsplan. Soll eine Verbesserung der Bewegungskompensation der Bestrahlung erreicht werden, kann in dem fünften Schritt 55b die Verbesserung des Scanpfads 24A bis 24D ausgewählt werden.

In dem sechsten Schritt 56b werden die Zielpunkte 30 zu Teilmengen zugeordnet, also gruppiert. In einem siebten Schritt 57b werden Teilbestrahlungspläne erzeugt und die Zielpunkte 30 den Teilbestrahlungsplänen zugeordnet, wobei aus jeder Gruppe zumindest ein Zielpunkt 30 zu einem Teilbestrahlungsplan zugeordnet wird.

Die einzelnen Scanpfade 24A bis 24D werden in einem achten Schritt 58b für jeden einzelnen Teilbestrahlungsplan festgelegt und in einem neunten Schritt 59b die Teilbestrahlungspläne derart zusammengefasst, dass die Energie des Partikelstrahls 20 möglichst selten variiert werden muss, das heißt, es werden die Zielpunkte 30 einer Isoenergieschicht 341 bis 347 zusammengefasst und alle Teilbestrahlungspläne ergeben gemeinsam einen übergeordneten Bestrahlungsplan.

Der Bestrahlungsplan wird in einem zehnten Schritt 60 schließlich an das Beschleunigerkontrollsystem zum Einstellen der Beschleunigerparameter übermittelt.

Fig. 10c zeigt eine weiteres Ablaufdiagramm, wobei in einem ersten Schritt 51c die Patientendaten geladen werden. In einem zweiten Schritt 52c werden die Optimierungsparameter gesetzt, wobei die Zielpunkte 30 definiert werden und ein engerer Punktabstand der Zielpunkte 30 zueinander gesetzt wird, beispielsweise 1 mm. Des Weiteren wird der Abstand von Isoenergieschichten festgelegt und verkürzt, beispielsweise auf 1 mm.

Hierbei wird in einem dritten Schritt 53b überprüft, dass möglichst wenige Zielpunkte 30 des Rasters bei der Optimierung der Dosisverteilungsberechnung nicht angefahren werden. Mit anderen Worten soll die Dosis auf möglichst viele Zielpunkte 30 des Zielvolumens 34 verteilt werden. Darüber hinaus wird auch überprüft, ob benachbarte Rasterpunkte eine ähnliche Anzahl von Partikeln erhalten und somit die Homogenität der Dosisverteilung weiter gesteigert werden kann.

Die Optimierung führt schließlich in einem vierten Schritt 54c zu einem Bestrahlungsplan. Soll eine Verbesserung der Bewegungskompensation der Bestrahlung erreicht werden, kann in dem fünften Schritt 55c die Verbesserung des Scanpfads 24A bis 24D ausgewählt werden.

In dem sechsten Schritt 56c werden die Zielpunkte 30 zu Teilmengen zugeordnet, also gruppiert, wobei auch berücksichtigt wird, dass eine Gruppe Punkte verschiedener Energieniveaus im 3D-Volumen enthält. Hierbei werden also Zielpunkte 30 berücksichtigt, die sich nicht mit gleicher Eindringtiefe des Partikelstrahls 20 erreichen lassen, also nicht auf einem Isoenergieniveau liegen. Mit anderen Worten hat ein Zielgebiet 32 nicht nur Punkte einer Isoenergieschicht 345, sondern auch die benachbarten Punkte der benachbarten Isoenergieschichten 344 bzw. 346, so dass sich die Anzahl der Zielpunkte 30 pro Zielgebiet 32 erhöht. Demgemäß ist ein Zielgebiet 32 ein 3D-Zielgebiet 32 und enthält, beispielsweise in einem kugelförmigen Bereich, Zielpunkte 30 mit einem Punktabstand auch in Strahlrichtung.

In einem siebten Schritt 57c werden Teilbestrahlungspläne erzeugt und die Zielpunkte 30 den Teilbestrahlungsplänen zugeordnet, wobei aus jeder Gruppe zumindest ein Zielpunkt 30 zu einem Teilbestrahlungsplan zugeordnet wird. Da das Zielvolumen 34 nun ggf. volumetrisch abgetastet wird, können auch schnelle Energievariationen des Partikelstrahls 20 zu berücksichtigen sein. Darüber hinaus werden die einzelnen Scanpfade 24A bis 24D für jeden einzelnen Teilbestrahlungsplan festgelegt.

In einem achten Schritt 58c werden die Teilbestrahlungspläne zusammengefasst und der übergeordnete Bestrahlungsplan erzeugt. Schließlich wird in einem letzten Schritt 60 der Bestrahlungsplan an das Beschleunigerkontrollsystem zum Einstellen der Beschleunigerparameter übermittelt.

Es ist dem Fachmann ersichtlich, dass die vorstehend beschriebenen Ausführungsformen beispielhaft zu verstehen sind, und die Erfindung nicht auf diese beschränkt ist, sondern in vielfältiger Weise variiert werden kann, ohne die Erfindung zu verlassen. Ferner ist ersichtlich, dass die Merkmale unabhängig davon, ob sie in der Beschreibung, den Ansprüchen, den Figuren oder anderweitig offenbart sind auch einzeln wesentliche Bestandteile der Erfindung definieren, selbst wenn sie zusammen mit anderen Merkmalen gemeinsam beschrieben sind.

### Bezugszeichenliste

- 10: Bestrahlungsanlage
- 11: Ionenquelle
- 12: Niedrigenergiestrahlführung
- 13: Vorbeschleuniger
- 15: Beschleunigereinheit
- 17: Strahlführung
- 19: Messraum
- 20: Partikelstrahl
- 21: Therapieraum
- 22: Ablenk- und Modulationseinrichtung
- 24, 24A, 24B, 24C, 24D: Scanpfad
- 30: Zielpunkt
- 32: Zielgebiet
- 34: Zielvolumen
- 36: Pfeil
- 40, 42: Scannermagnete zur lateralen Ablenkung des Partikelstrahls
- 44: Doppelkeilsystem zur longitudinalen Ablenkung (=Abbremsung) des Partikelstrahls
- 46: Bewegungserfassungseinrichtung
- 51a bis 51c: erster Schritt
- 52a bis 52c: zweiter Schritt
- 53a bis 53c: dritter Schritt
- 54a bis 54c: vierter Schritt
- 55a bis 55c: fünfter Schritt
- 56a bis 56c: sechster Schritt
- 57a bis 57c: siebter Schritt
- 58a bis 58c: achter Schritt
- 59a bis 59b: neunter Schritt
- 60: Abschlussschritt
- 71: Computer- oder Kernspintomograph
- 72: PET-Kamera
- 73: Abstandssensor
- 77: Körper
- 78: Lagerfläche
- 81: Vorrichtung zum Erstellen eines Datensatzes
- 82: Einrichtung zur quantitativen Erfassung von Bewegungen
- 86: Steuerung
- 87: Steuerleitung
- A bis D: Teilmenge an Zielpunkten

## Patentansprüche

1. Verfahren zur Bestrahlungsplanung eines Zielvolumens (34) mit einem gescannten Partikelstrahl (20), umfassend die Schritte:
- Festlegen eines in einem Körper (77) angeordneten Zielvolumens (34),
- Unterteilen des Zielvolumens (34) in eine Vielzahl einzeln anfahrbarer Zielpunkte (30),
- Festlegen einer Anzahl von zeitlich konsekutiven Teilbestrahlungsplänen,
**dadurch gekennzeichnet,**
**dass** es den Schritt umfasst:
- Aufteilen der Zielpunkte (30) des Zielvolumens (34) in Teilmengen (A, B, C, D) auf die Teilbestrahlungspläne, wobei die Teilmengen (A, B, C, D) jeweils über das gesamte Zielvolumen (34) verteilt sind und wobei zueinander benachbarte Zielpunkte (30) des Zielvolumens (34) jeweils unterschiedlichen Teilbestrahlungsplänen zugeordnet werden.

2. Steuerungsvorrichtung für eine Bestrahlungsanlage (10), die einen gescannten Partikelstrahl (20) bereitstellt, mit
einer Steuerungseinheit (86), die die Bestrahlungsanlage bei einer Bestrahlung steuert, um Zielpunkte (30) eines in einem Körper (77) angeordneten Zielvolumens (34) mit dem gescannten Partikelstrahl (20) anzufahren,
wobei die Zielpunkte in Teilmengen (A, B, C, D) auf zeitlich konsekutiv abzuarbeitende Teilbestrahlungspläne aufgeteilt sind, **dadurch gekennzeichnet, dass** die Teilmengen jeweils über das gesamte Zielvolumen verteilt sind und
wobei zueinander benachbarte Zielpunkte zu jeweils unterschiedlichen Teilmengen zugeordnet sind.

3. Steuerungsvorrichtung nach dem vorstehenden Anspruch,
wobei die Zielpunkte (30) des Zielvolumens (34) örtlich alternierend den Teilmengen (A, B, C, D) zugeordnet sind und/oder
wobei die Zielpunkte (30) der Teilmengen (A, B, C, D) jeweils nur von Zielpunkten benachbart umgeben sind, die den anderen Teilmengen zugeordnet sind und/oder
wobei die Zielpunkte (30) zeilen- und/oder spaltenweise schachbrettartig alternierend den unterschiedlichen Teilmengen (A, B, C, D) zugeordnet sind.

4. Steuerungsvorrichtung nach einem der Ansprüche 2 oder 3,
wobei die Steuerungseinheit (86) ausgebildet ist, die Zielpunkte (30) des Zielvolumens (34) derart mit dem gescannten Partikelstrahl (20) anzufahren, dass innerhalb der Teilbestrahlungspläne benachbarte Zielpunkte (30) des Zielvolumens (34) übersprungen werden und/oder
wobei die Steuerungseinheit (86) ausgebildet ist, die Zielpunkte (30) von zumindest zwei Teilmengen (A, B, C, D) mäanderförmig miteinander verschränkt anzufahren.

5. Steuerungsvorrichtung nach einem der Ansprüche 2 bis 4,
wobei jeder Zielpunkt (30) des Zielvolumens (34) jeweils genau einer Teilmenge (A, B, C, D) zugeordnet ist.

6. Steuerungsvorrichtung nach einem der Ansprüche 2 bis 5,
wobei die Steuerungseinheit (86) die Bestrahlungsanlage (10) derart steuert, benachbarte Zielpunkte des Zielvolumens (34) nicht unmittelbar zeitlich aufeinander folgend anzufahren.

7. Steuerungsvorrichtung nach einem der Ansprüche 2 bis 6,
wobei die Anzahl der jeweils in einem gemeinsamen Zielgebiet (32) angeordneten Zielpunkte (30) der Anzahl der Teilbestrahlungspläne entspricht, wobei die Zielgebiete (32) des Zielvolumens (34) jeweils dem erwarteten Strahldurchmesser des gescannten Partikelstrahls (20) entsprechen.

8. Steuerungsvorrichtung nach einem der Ansprüche 2 bis 7,
wobei die jeweils in einem gemeinsamen Zielgebiet (32) angeordneten Zielpunkte (30) unterschiedlichen Teilmengen (A, B, C, D) zugeordnet sind, wobei die Zielgebiete (32) des Zielvolumens (34) jeweils dem erwarteten Strahldurchmesser des gescannten Partikelstrahls (20) entsprechen.

9. Steuerungsvorrichtung nach einem der Ansprüche 2 bis 8,
wobei die in einem gemeinsamen Zielgebiet (32) angeordneten Zielpunkte (30) jeweils genau einer Teilmenge (A, B, C, D) zugeordnet sind, wobei die Zielgebiete des Zielvolumens (34) jeweils dem erwarteten Strahldurchmesser des gescannten Partikelstrahls (20) entsprechen.

10. Steuerungsvorrichtung nach einem der Ansprüche 2 bis 9,
wobei die Steuerungseinheit (86) die Bestrahlungsanlage (10) derart steuert, von den in einem gemeinsamen Zielgebiet (32) angeordneten Zielpunkten (30) mit den Teilbestrahlungsplänen höchstens jeweils einen Zielpunkt anzufahren.

11. Steuerungsvorrichtung nach einem der Ansprüche 2 bis 10,
wobei die Zielpunkte (30) des Zielvolumens (34) in Isoenergieschichten (341 bis 347) angeordnet sind und wobei jede Isoenergieschicht Zielpunkte von mehreren Teilmengen (A, B, C, D) umfasst.

12. Steuerungsvorrichtung nach einem der Ansprüche 2 bis 11,
wobei die Zielpunkte (30) des Zielvolumens (34) in Isoenergieschichten (341 bis 347) angeordnet sind und
wobei die Steuerungseinheit (86) die Bestrahlungsanlage (10) derart steuert, die Zielpunkte zumindest einer der Teilmengen (A, B, C, D) in den Isoenergieschichten jeweils konsekutiv anzufahren oder
wobei die Steuerungseinheit (86) die Bestrahlungsanlage (10) derart steuert, die Zielpunkte zumindest einer der Teilmengen (A, B, C, D) in den Isoenergieschichten jeweils abwechselnd zeilenweise und spaltenweise anzufahren.

13. Steuerungsvorrichtung nach einem der Ansprüche 2 bis 12,
wobei die Zielpunkte (30) des Zielvolumens (34) in Isoenergieschichten (341 bis 347) angeordnet sind und
wobei die Steuerungseinheit (86) die Bestrahlungsanlage (10) derart steuert, innerhalb der Teilbestrahlungspläne jeweils jede zweite Zeile oder jede zweite Spalte der Zielpunkte des Zielvolumens zu überspringen.

14. Steuerungsvorrichtung nach einem der Ansprüche 2 bis 13,
wobei für die Festlegung der Anzahl von Teilbestrahlungsplänen die Anzahl der Zielgebiete (32), die lokalen Bewegungsparameter und/oder eine vorberechnete Vordosis, die durch das vorherige Anfahren anderer Zielpunkte (30) entsteht, berücksichtigt wird.

15. Bestrahlungsanlage mit einer Steuerungsvorrichtung nach einem der Ansprüche 2 bis 14.

## Claims

1. Method for irradiation planning of a target volume (34) with a scanned particle beam (20), comprising the following steps:
- defining a target volume (34) located in a body (77),
- subdividing the target volume (34) into a plurality of individually approachable target points (30),
- defining a number of temporally consecutive irradiation sub-plans,
**characterized in that** it comprises the step:
- dividing the target points (30) of the target volume (34) among the irradiation sub-plans in subsets (A, B, C, D), wherein the subsets (A, B, C, D) are each distributed over the entire target volume (34), and wherein mutually adjacent target points (30) of the target volume (34) are assigned to different irradiation sub-plans.

2. Control device for a radiation facility (10) that provides a scanned particle beam (20), having
a control unit (86) that controls the radiation facility during irradiation in order to approach target points (30) of a target volume (34) located in a body (77) with the scanned particle beam (20),
wherein the target points (30) are divided among irradiation sub-plans in subsets (A, B, C, D), the irradiation sub-plans to be executed consecutively in time,
**characterized in that**
the subsets are each distributed over the entire target volume, and
wherein mutually adjacent target points are assigned to different subsets.

3. Control device according to the preceding claim,
wherein the target points (30) of the target volume (34) are assigned to the subsets (A, B, C, D) in spatial alternation, and/or
wherein each of the target points (30) of the subsets (A, B, C, D) is contiguously surrounded only by target points that are assigned to the other subsets, and/or
wherein the target points (30) are assigned to the different subsets (A, B, C, D) in alternation within rows and/or columns in checkerboard style.

4. Control device according to one of claims 2 or 3,
wherein the control unit (86) is configured to approach the target points (30) of the target volume (34) with the scanned particle beam (20) in such a way that adjacent target points (30) of the target volume (34) within the irradiation sub-plans are skipped, and/or
wherein the control unit (86) is configured to approach the target points (30) of at least two subsets (A, B, C, D) interlaced with one another in a meander pattern.

5. Control device according to one of claims 2 to 4,
wherein each target point (30) of the target volume (34) is assigned to exactly one subset (A, B, C, D).

6. Control device according to one of claims 2 to 5,
wherein the control unit (86) controls the radiation facility (10) in such a way that it does not approach adjacent target points of the target volume (34) in direct temporal sequence.

7. Control device according to one of claims 2 to 6,
wherein the number of target points (30) located in a common target area (32) corresponds to the number of irradiation sub-plans, wherein the target areas of the target volume (34) each correspond to the anticipated beam diameter of the scanned particle beam (20).

8. Control device according to one of claims 2 to 7,
wherein the target points (30) located in a common target area (32) are each assigned to different subsets (A, B, C, D), wherein the target areas of the target volume (34) each correspond to the anticipated beam diameter of the scanned particle beam (20).

9. Control device according to one of claims 2 to 8,
wherein the target points (30) located in a common target area (32) are each assigned to exactly one subset (A, B, C, D), wherein the target areas of the target volume (34) each correspond to the anticipated beam diameter of the scanned particle beam (20).

10. Control device according to one of claims 2 to 9,
wherein the control unit (86) controls the radiation facility (10) so as to approach a maximum of one target point, of the target points (30) located in a common target area (32), with each of the irradiation sub-plans.

11. Control device according to one of claims 2 to 10,
wherein the target points (30) of the target volume (34) are arranged in iso-energy layers (341 to 347), and wherein each iso-energy layer includes target points from multiple subsets (A, B, C, D).

12. Control device according to one of claims 2 to 11,
wherein the target points (30) of the target volume (34) are arranged in iso-energy layers (341 to 347), and
wherein the control unit (86) controls the radiation facility (10) so as to approach the target points of at least one of the subsets (A, B, C, D) in the iso-energy layers consecutively, or
wherein the control unit (86) controls the radiation facility (10) so as to approach the target points of at least one of the subsets (A, B, C, D) in the iso-energy layers in alternation by rows and columns.

13. Control device according to one of claims 2 to 12,
wherein the target points (30) of the target volume (34) are arranged in iso-energy layers (341 to 347), and
wherein the control unit (86) controls the radiation facility (10) so as to skip every other row or every other column of target points of the target volume within the irradiation sub-plans.

14. Control device according to one of claims 2 to 13,
wherein the number of target areas (32), the local motion parameters, and/or a pre-calculated pre-dose that occurs as a result of the prior approach to other target points (30), are taken into account for defining the number of irradiation sub-plans.

15. Radiation facility having a control device according to one of the claims 2 to 14.

## Revendications

1. Procédé permettant d'établir des plans d'irradiation d'un volume cible (34) avec un faisceau de particules (20) balayé, comprenant les étapes suivantes:
- définition d'un volume cible (34) disposé dans un corps (77),
- division du volume cible (34) en une multitude de points cibles (30) pouvant être sélectionnés individuellement,
- définition d'un nombre de plans d'irradiation partiels consécutifs dans le temps,
**caractérisé en ce qu'**il comprend l'étape suivante:
- répartition des points cibles (30) du volume cible (34) en sous-ensembles (A, B, C, D) sur les plans d'irradiation partiels, sachant que les sous-ensembles (A, B, C, D) sont répartis respectivement sur la totalité du volume cible (34) et des points cibles (30), mutuellement voisins, du volume cible (34) sont associés respectivement à des plans d'irradiation partiels différents.

2. Dispositif de commande pour une installation d'irradiation (10) qui fournit un faisceau de particules (20) balayé, comprenant
une unité de commande (86) qui commande l'installation d'irradiation lors d'une irradiation, afin de sélectionner, avec le faisceau de particules (20) balayé, des points cibles (30) d'un volume cible (34) disposé dans un corps (77),
les points cibles étant répartis en sous-ensembles (A, B, C, D) sur des plans d'irradiation partiels à exécuter successivement dans le temps, **caractérisé en ce que** les sous-ensembles sont répartis respectivement sur la totalité du volume cible et
des points cibles mutuellement voisins étant associés à des sous-ensembles respectifs différents.

3. Dispositif de commande selon la revendication précédente,
dans lequel les points cibles (30) du volume cible (34) sont associés localement en alternance aux sous-ensembles (A, B, C, D) et/ou
dans lequel les points cibles (30) des sous-ensembles (A, B, C, D) sont respectivement entourés uniquement de points cibles voisins qui sont associés aux autres sous-ensembles et/ou
dans lequel les points cibles (30) sont associés aux différents sous-ensembles (A, B, C, D) en alternance par lignes et/ou par colonnes, sous forme d'échiquier.

4. Dispositif de commande selon l'une des revendications 2 ou 3,
dans lequel l'unité de commande (86) est agencée pour sélectionner les points cibles (30) du volume cible (34) avec le faisceau de particules (20) balayé, de manière telle que, à l'intérieur des plans d'irradiation partiels, des points cibles (30) voisins appartenant au volume cible (34) soient omis et/ou
dans lequel l'unité de commande (86) est agencée pour sélectionner les points cibles (30) d'au moins deux sous-ensembles (A, B, C, D) de façon croisée en méandres.

5. Dispositif de commande selon l'une des revendications 2 à 4,
dans lequel chaque point cible (30) du volume cible (34) est associé respectivement à exactement un sous-ensemble (A, B, C, D).

6. Dispositif de commande selon l'une des revendications 2 à 5,
dans lequel l'unité de commande (86) commande l'installation d'irradiation (10) de manière à ce qu'elle ne sélectionne pas directement successivement dans le temps des points cibles voisins appartenant au volume cible (34).

7. Dispositif de commande selon l'une des revendications 2 à 6,
dans lequel le nombre des points cibles (30) disposés respectivement dans une zone cible (32) commune correspond au nombre de plans d'irradiation partiels, sachant que les zones cibles (32) du volume cible (34) correspondent respectivement au diamètre de faisceau prévu du faisceau de particules (20) balayé.

8. Dispositif de commande selon l'une des revendications 2 à 7,
dans lequel les points cibles (30) disposés respectivement dans une zone cible (32) commune sont associés à des sous-ensembles (A, B, C, D) différents, sachant que les zones cibles (32) du volume cible (34) correspondent respectivement au diamètre de faisceau prévu du faisceau de particules (20) balayé.

9. Dispositif de commande selon l'une des revendications 2 à 8,
dans lequel les points cibles (30) disposés dans une zone cible (32) commune sont associés respectivement à exactement un sous-ensemble (A, B, C, D), sachant que les zones cibles du volume cible (34) correspondent respectivement au diamètre de faisceau prévu du faisceau de particules (20) balayé.

10. Dispositif de commande selon l'une des revendications 2 à 9,
dans lequel l'unité de commande (86) commande l'installation d'irradiation (10) de manière à ce qu'elle sélectionne, avec les plans d'irradiation partiels, au maximum respectivement un point cible parmi les points cibles (30) disposés dans une zone cible (32) commune.

11. Dispositif de commande selon l'une des revendications 2 à 10,
dans lequel les points cibles (30) du volume cible (34) sont disposés dans des couches d'isoénergie (341 à 347) et dans lequel chaque couche d'isoénergie comprend des points cibles de plusieurs sous-ensembles (A, B, C, D).

12. Dispositif de commande selon l'une des revendications 2 à 11,
dans lequel les points cibles (30) du volume cible (34) sont disposés dans des couches d'isoénergie (341 à 347) et
dans lequel l'unité de commande (86) commande l'installation d'irradiation (10) de manière à ce qu'elle sélectionne respectivement de façon consécutive les points cibles d'au moins un des sous-ensembles (A, B, C, D) dans les couches d'isoénergie ou
dans lequel l'unité de commande (86) commande l'installation d'irradiation (10) de manière à ce qu'elle sélectionne respectivement en alternance par lignes et par colonnes les points cibles d'au moins un des sous-ensembles (A, B, C, D) dans les couches d'isoénergie.

13. Dispositif de commande selon l'une des revendications 2 à 12,
dans lequel les points cibles (30) du volume cible (34) sont disposés dans des couches d'isoénergie (341 à 347) et
dans lequel l'unité de commande (86) commande l'installation d'irradiation (10) de manière à ce que, à l'intérieur des plans d'irradiation partiels, elle omette une ligne sur deux ou une colonne sur deux des points cibles du volume cible.

14. Dispositif de commande selon l'une des revendications 2 à 13,
dans lequel, pour la définition du nombre de plans d'irradiation partiels, on prend en compte le nombre des zones cibles (32), les paramètres de mouvement locaux et/ou une prédose précalculée, qui est obtenue par la sélection préalable d'autres points cibles (30).

15. Installation d'irradiation comprenant un dispositif de commande selon l'une des revendications 2 à 14.
